# EUROPEAN PATENT APPLICATION

(11) **EP 4 570 166 A1**
(43) Date of publication of application: **18.06.2025**
(21) Application number: 24219336.5
(22) Date of filing: 12.12.2024
(51) Int. Cl.: A61B 3/14, G01J 1/04, G01N 21/76, H01J 31/50

(54) **SYSTEM FOR PHOTOMULTIPLIER PROTECTION IN EYE IMAGING**

(30) Priority: 12.12.2023 EP 23461688
(71) Applicant: Instytut Chemii Fizycznej Polskiej Akademii Nauk, 01-224 Warszawa (PL)
(72) Inventor: WOJTKOWSKI, Maciej, 01-224 Warszawa (PL)
(74) Representative: AOMB Polska Sp. z.o.o.

(57) **Abstract**

The invention elates to a system for protecting photomultiplier in eye imaging comprising an optical measuring system (1), comprising a light level detector (13) in a vicinity of a photomultiplier (11) which is protected by means for blocking the input aperture (14), the optical measuring system being connected in an airtight manner to a front side (21) of the non-transparent head chamber (2) with airtight fastening of the bottom side (22) around patient's jaw, neck or arms, wherein the optical measuring system also comprises a chinrest (15) and the light level detector (13) is preset with maximum acceptable threshold value, above which the means for blocking the input aperture (14) block the light on the photomultiplier.

## Description

### Technical field

The invention is related to the field of ophthalmology, especially eye imaging using a photomultiplier.

### Background

Since many eye diseases are progressive, early diagnostic is crucial before the disease reaches a point when it becomes irreversible. Early detecting of symptoms increases chances of proper treatment, which makes it possible to stop or reduce the progression of the disease. Due to the need of non-invasive functional imaging growing interest in the ophthalmology is addresses to the two-photon (2Ph) fluorescence.

In the context of measurements of the in-vivo two-photon fluorescence from the eye typically the detected signal levels are very low. The reason is a need for maintaining the safe eye illumination intensity level to avoid any potential eye damage and, as a consequence, low intensity of the fluorescence emitted by the tissue. In order to capture the fluorescence light by the measurement system efficiently, typically a high-gain sensitive light detectors are used, for example photomultipliers.

A photomultiplier is a very light-sensitive device and this property is achieved by its design with the application of high voltage to induce a detectable signal for even single-photon absorption events. The drawback in this approach is a danger or damaging the photomultiplier in a situation of relatively high light intensity, which causes too high amplification of the signal and appearance of excessive current which destroys the electronic components of the detector. To avoid this, it is sufficient to cut the high voltage power supply from the photomultiplier before the electron flow builds up.

In the human eye measurement, the platform consists of a typical chin rest known from standard commercial ophthalmic devices. Due to the light detector sensitivity, the measurements are performed in total darkness to avoid any damage to the electronics. It would be beneficial to introduce a measurement protocol which allows for conducting measurements in ambient light conditions. However, the optical system inevitably guides any excess ambient light to the detector, causing its immediate destruction.

US8989848B2 discloses an apparatus for determining characteristics of biological tissue or other medium of a subject, comprising: a) means for illuminating the medium with electromagnetic radiation directed into the medium, the means for illuminating comprising a blue LED light source, thereby causing the medium to react with a first action selected from the group consisting of reflecting, backscattering, transmitting, and emitting responsive radiation and a second action selected from the same group excluding the first action, b) a detector for collecting the responsive radiation, c) an optical filter connected to the detector, for separating the collected radiation into a plurality of components, wherein the plurality of components comprises a fluorescent component, d) a processor operable to (i) measure the intensity of each of the separated plurality of components and (ii) determine a mathematical relationship between the separated plurality of components, e) a LED fixation tube for guiding the subject to self-align itself along an axis, f) a lens, optically responsive to the light from the light source, for focusing the light; and g) a lens system, optically responsive to the focused light, having a focus, and defining an aperture at its focus of less than approximately 1 cm, wherein the wavelength of the fluorescent component of the radiation is selected from the group consisting of between approximately 465-500 nm and 520-600 nm.

US9451909B2 discloses a method for detecting an amyloid protein in an eye of a mammal, the method comprising: illuminating the eye with a light source having at least one of a wavelength property, a polarization property or a combination thereof, each appropriate to produce fluorescence in at least an amyloid-binding compound when the amyloid-binding compound is bound to the amyloid protein, the amyloid-binding compound having been introduced to the eye and specifically binding to the amyloid protein indicative of the amyloidogenic disorder; receiving light including fluorescence produced as a result of the illuminating the eye; and determining a time decay rate of fluorescence for at least the fluorescence produced by the amyloid-binding compound bound to the amyloid protein, the determining permitting distinguishing of the presence of the amyloid-binding compound bound to the amyloid protein in the eye based on at least the time decay rate, the determining comprising performing a time correlation single photon counting of fluorescence produced by the eye; wherein the distinguishing the presence of the amyloid-binding compound bound to the amyloid protein comprises distinguishing the amyloid-binding compound bound to the amyloid protein from background autofluorescence of eye tissues, autofluorescence of other non-specific particles and unbound amyloid-binding compound.

PL235165 discloses a method of protecting the photocathode of a photomultiplier of a detection unit with an exchangeable scintillation chamber from unwanted light, consisting in moving a movable shutter in a cyclic manner, the position in relation to the photomultiplier window being determined by the rotational movement of the exchangeable scintillation chamber associated with its insertion and removal, from the detector unit socket in such a way that, in the insertion/removal position, the photocathode of the photomultiplier is shielded from external light, while, during rotation of the scintillation cell to the measurement position, the diaphragm is moved, in parallel, to a position where it does not obstruct the photocathode. To remove the scintillation chamber from the detector assembly slot, the scintillation chamber must be rotated again to the chamber insertion/removal position. The prior art document also discloses a device for preventing unwanted illumination of the photocathode of a photomultiplier of a detection assembly with a removable scintillation chamber, characterised in that it is a movable ring 2 on the lower surface of which are two pins 4 and on the upper surface of which are a vertical rod 5 fixed perpendicularly to the surface of this ring. The pins 4 on the lower surface of the ring couple the ring to the scintillation chamber, and the leader 5 of the movable ring is placed in the longitudinal opening 7 of the movable flat aperture 6, coupling it to the drive mechanism of the scintillation chamber.

It is therefore advisable to further develop methods and devices that are able to efficiently protect photomultiplier from being damaged during the measurement of the in-vivo two-photon fluorescence from the eye without the need to operate the system in a darkroom.

**The** object of the present invention is a system which automatically protects the photomultiplier from getting damaged by too high level of incoming light in the in vivo eye measurement of two-photon fluorescence, making it possible to perform measurements with typical levels of room ambient light.

### Summary of the invention

The invention provides a system for protecting photomultiplier in eye imaging comprising an optical measuring system, comprising a light level detector in a vicinity of a photomultiplier which is protected by means for blocking the input aperture, the optical measuring system being connected in an airtight manner to a front side of the non-transparent head chamber with airtight fastening of the bottom side around patient's jaw, neck or arms, wherein the optical measuring system also comprises a chinrest and the light level detector is preset with maximum acceptable threshold value, above which the means for blocking the input aperture block the light on the photomultiplier.

**Preferably,** the airtight connection of the front side and the bottom side of the head chamber is formed of a soft matter, preferably inflatable.

Preferably, the optical measuring system and the head chamber are separate constructions.

Preferably, the optical measuring system and the head chamber are connected with mechanical hinges.

**Preferably,** connection between the optical measuring system and the head chamber is secured using a fastener 5.

**Preferably,** means for blocking the input aperture is shutter, preferably fast shutter.

Preferably, the head chamber comprises ventilation ports equipped with input and output fans.

**Preferably,** ventilation ports are separated from the outside environment by a filter, preferably HEPA type filter.

Preferably, light level detector monitors the intensity of the control beam (CB) split from the signal beam (SB).

### Description of figures

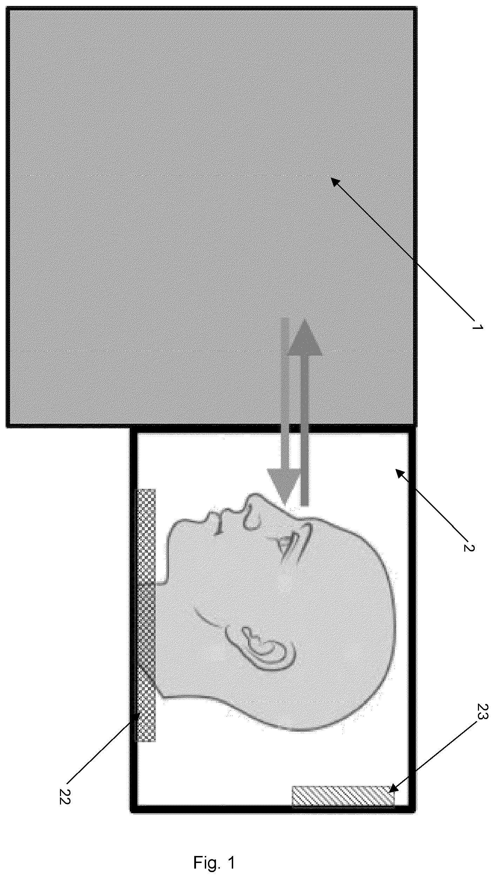

Fig. 1 presents schematic of the arrangement of the patient, helmet and the optical measurement system. No ambient light can enter the inside of the helmet during the measurement to protect the photomultiplier inside the optical measurement system

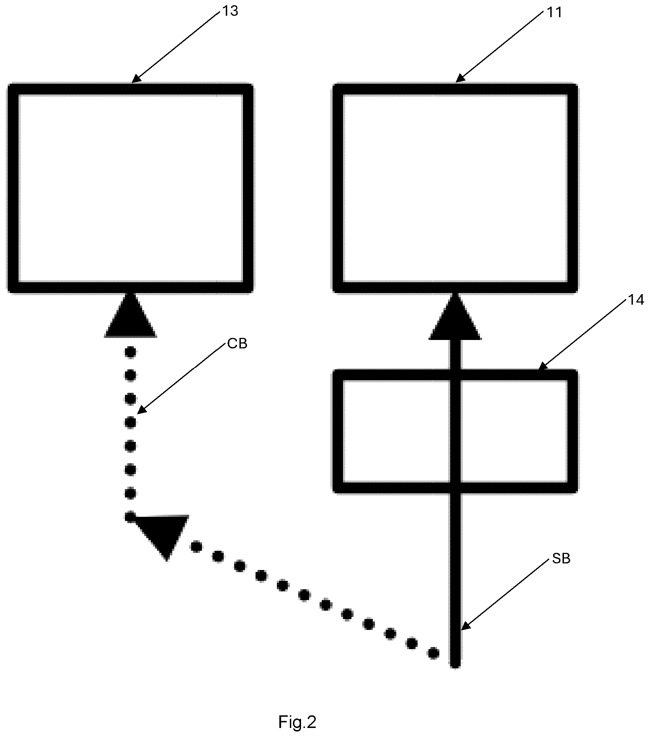

Fig. 2 presents block diagram of the photomultiplier protection system inside the optical measurement system. The control beam CB illuminates a calibrated photodetector which has a preset maximum light intensity level. Immediately after this level is exceeded, the beam shutter is closed to block the light on the photomultiplier

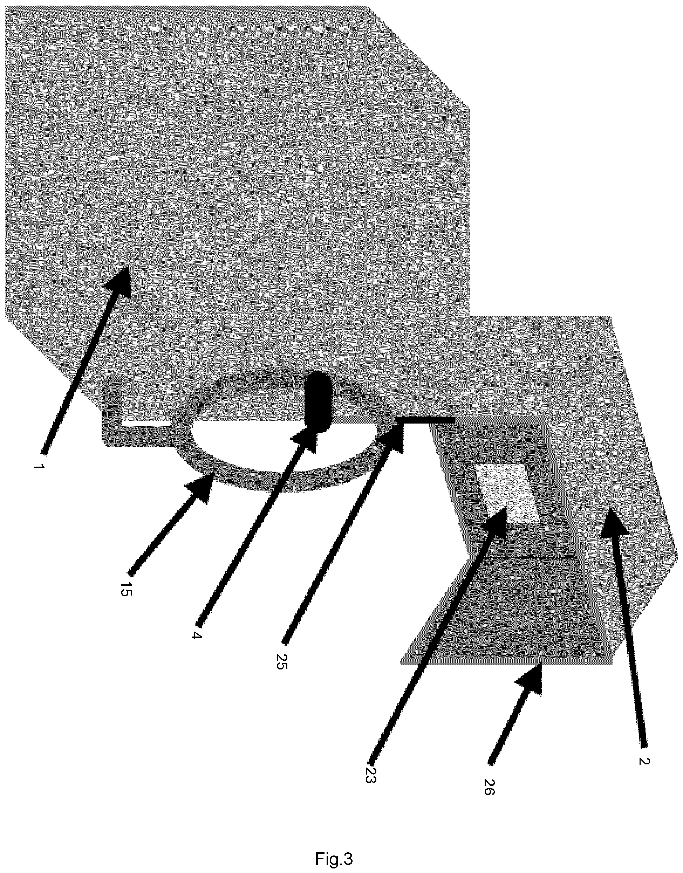

Fig. 3 presents embodiment in which the head chamber 2 is connected to the imaging device using side hinges, shown in an open position
Fig. 4 presents a system of the invention with the head chamber 2 closed for the measurement.

The following designations are used in the attached drawing: 1 - optical measuring system; 2 - head chamber; 3 - patient's head; 4 - objective; 5 - fastener; 11 - photomultiplier; 13 - light level detector; 14 - input aperture; 15 - chinrest; 21 - front of the imaging device; 22 - bottom side of the head chamber; 23 - ventilation ports; 24a - input fans; 24b - output fans; 25 - mechanical hinges; 26 - airtight connection.

### Detailed description of invention

The system according to the present invention is based on three main subcom ponents:
automatic detection of the light level in the vicinity of the photomultiplier 11 with a preset maximum acceptable threshold value,
patient's head chamber which separates the room light from the dark part of the optical system, and
automatic fast shutter as means for blocking the input aperture 14 capable of blocking the input aperture of the light level detector 13.

The light level detector 13 can be placed anywhere inside the optical system or inside the head chamber, however it is preferred to position the detector in the vicinity the photomultiplier 11 to achieve the most accurate measurement of the light level on the photomultiplier 11. An alternative approach is to split the detected beam and use part of its intensity to monitor the light level. Another method would be using parasitic or deliberate reflection found or arranged in the optical system and monitoring the light level with it.

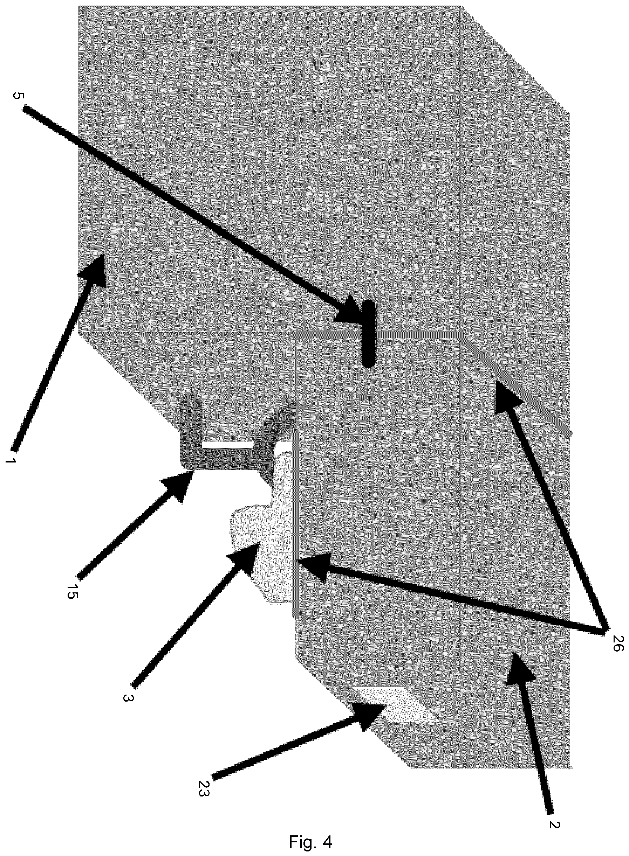

As shown in the Fig. 4 patient's head chamber needs to be lightweight, tightly blocking the light around the patient's jaw, neck or arms and ventilated, at least passively, allowing the patient to breathe freely. In the preferred embodiment, the head chamber is ventilated by using input and output fans connected to the ventilation ports 23, for example through a HEPA filter or any similar barrier blocking the light. The helmet needs to be integrated in a continuous way with the measurement system to block the ambient light on the interface between the helmet and the measurement system.

Commercially available fast shutters for eye safety are optimized for speed and used in the configuration of being always powered when open. In the case of their power supply drop for any reason such shutters close fast enough to block the light beam and minimize a danger of causing an eye damage this way. This property could be used to protect the photomultiplier 11 in a situation when an excess light intensity on the light level detector 13 is recorded. In the context of the present invention a fast shutter should be interpreted as a shutter that has a closing time below 30 ms.

The advantages of the system for automatic protection of the photomultiplier 11 from getting damaged by excess light:
Avoiding the photomultiplier 11 damage by accidental increase of the ambient light level during eye measurements or as a result of the measurement system misuse Possibility of performing the two-photon fluorescence in vivo eye measurements with the room ambient light switched on, making it compatible with typical ophthalmic practice procedures.

The chamber is prepared in a form of a box with its upper, side and back walls solid. The front 21 and the bottom 22 sides are formed of a soft matter which is capable of being tightly attached to the front 21 of the imaging device and the patient's head, for example by means of pumping the air into an inflatable interface. The chamber may be attached to the imaging device with mechanical hinges or be a separate construction, attached to the imaging device only for the time of measurement. In a preferred embodiment a connection between the optical measuring system and the head chamber is additionally secured using a fastener 5

**The** head chamber 2 is made of non-transparent materials to avoid external illumination light penetrating into the chamber.

To avoid light penetrating through the connection between the head chamber 2 and the optical measuring system 1 and the patient's head, it is essential for these connections to be airtight.

For the needs of the measurement, the patient positions their head in a standard ophthalmic chinrest 15 and then the operator positions the chamber over the patient's head, either from a side using the hinges or slides it from the top if it is a separate item. Afterwards the space between the chamber and the imaging device is being secured against any light leaks, preferably by inflating a seal to fill any gaps. Also, the gaps between the chamber and the patient's head need to be closed in the same way, for example by inflating an airtight ring around the patient's back of the head and the jaw.

One of the sides of the head chamber further comprises at least one ventilation port 23, connected to an input fan and an output fan, preferably both separated from the outside environment with a suitable non-airtight filter, e.g. cloth (polyester or fiberglass including HEPA class), paper or the HEPA type. In the most preferred embodiment filter is HEPA type.

System according to the present invention is suitable for any optical measuring system comprising a photomultiplier.

## Claims

1. System for protecting photomultiplier in eye imaging comprising an optical measuring system (1), comprising a light level detector (13) in a vicinity of a photomultiplier (11) which is protected by means for blocking the input aperture (14), the optical measuring system being connected in an airtight manner to a front side (21) of the non-transparent head chamber (2) with airtight fastening of the bottom side (22) around patient's jaw, neck or arms, wherein the optical measuring system also comprises a chinrest (15) and the light level detector (13) is preset with maximum acceptable threshold value, above which the means for blocking the input aperture (14) block the light on the photomultiplier.

2. System according to claim 1, wherein airtight connection (26) of the front side (21) and the bottom side (22) of the head chamber (2) is formed of a soft matter, preferably inflatable.

3. System according to claims 1 and 2, wherein optical measuring system (1) and head chamber (2) are separate constructions.

4. System according to claims 1 and 2, wherein optical measuring system (1) and head chamber (2) are connected with mechanical hinges (25).

5. System according to claim 4, wherein a connection between the optical measuring system (1) and the head chamber (2) is secured using a fastener (5)

6. System according to claims 1-4, wherein means for blocking the input aperture (14) is shutter, preferably fast shutter.

7. System according to claims 1-5, wherein head chamber (2) comprises ventilation ports (23) equipped with input (24a) and output (24b) fans.

8. System according to claim 6, wherein ventilation ports are separated from the outside environment by a filter, preferably HEPA type filter.

9. System according to any of the preceding claims wherein light level detector (13) monitors the intensity of the control beam (CB) split from the signal beam (SB).
